(19) Europäisches Patentamt
European Patent Office
Office européen des brevets

(11) Publication number: **0 109 299 A2**

(12) **EUROPEAN PATENT APPLICATION**

(21) Application number: 83306935.4

(22) Date of filing: 14.11.83

(51) Int. Cl.³: **C 07 D 241/12**, C 07 D 213/30, C 07 D 239/26, C 07 D 409/06, A 01 N 43/40, A 01 N 43/54, A 01 N 43/60

(30) Priority: 16.11.82 US 442061

(43) Date of publication of application: 23.05.84 Bulletin 84/21

(84) Designated Contracting States: BE CH DE FR GB IT LI LU NL SE

(71) Applicant: **ELI LILLY AND COMPANY, 307, East McCarty Street, Indianapolis Indiana 46285 (US)**

(72) Inventor: **Krumkalns, Eriks Victor, 6333, North Rural, Indianapolis Indiana 46220 (US)**

(74) Representative: **Crowther, Terence Roger et al, Erl Wood Manor, Windlesham Surrey GU20 6PH (GB)**

(54) **1,1-Disubstituted-2-heterocyclic ethanol derivatives.**

(57) Use of a compound of the formula (I)

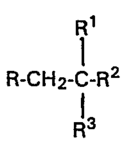

(I)

wherein:

R is 2-, 3- or 4-pyridyl, pyrazinyl, 3- or 4-pyridazinyl, or 2-, 4- or 5-pyrimidinyl;

$R^1$ is hydroxy, methoxy or ethoxy;

$R^2$ is $C_1$-$C_{10}$ alkyl, $C_3$-$C_8$ cycloalkyl, $C_1$-$C_4$ haloalkyl or

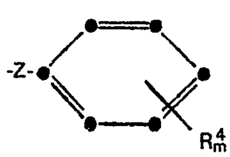

;

$R^3$ is $C_1$-$C_{10}$ alkyl, $C_3$-$C_8$ cycloalkyl, $C_3$-$C_8$ cycloalkyl bearing one or two substituents selected from the group consisting of $C_1$-$C_4$ alkyl, halogen, $C_1$-$C_4$ haloalkyl, and $C_1$-$C_4$ haloalkoxy, or $R^3$ is

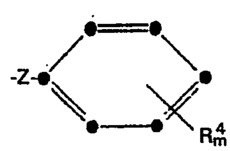

;

$R^4$ is halogen, $C_1$-$C_4$ alkyl, $C_1$-$C_4$ alkoxy, $C_1$-$C_4$ haloalkyl or $C_1$-$C_4$ haloalkoxy;

Z is $-CH_2-(CH_2)_m-O-$ or a direct link;

each m is independently 0, 1 or 2; or

$R^2$ and $R^3$, when taken together with the carbon atom to which they are attached, form dibenzo[A,D]cyclohepta[1,4]-dienylidene, 9-fluorenylidene, 9-thioxanthenylidene, 10,11-dihydro-5H-dibenzo[A,D]cyclohepten-5-ylidene or 1,2,3,4-tetrahydronaphthalen-1-ylidene;

or an agriculturally-ecceptable salt thereof as an antifugal agent or plant growth regulator.

EP 0 109 299 A2

# 1,1-DISUBSTITUTED-2-HETEROCYCLIC ETHANOL DERIVATIVES

The present invention relates to methods for inhibiting the growth of fungal diseases and controlling the growth of terrestrial and aquatic plants employing a compound of the formula (I):

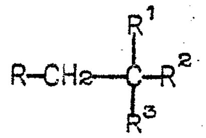

(I)

wherein:

R is 2-, 3- or 4-pyridyl, 2-pyrazinyl, 3- or 4-pyridazinyl, or 2-, 4- or 5-pyrimidinyl;

$R^1$ is hydroxy, methoxy or ethoxy;

$R^2$ is $C_1$-$C_{10}$ alkyl, $C_3$-$C_8$ cycloalkyl, $C_1$-$C_4$ haloalkyl or

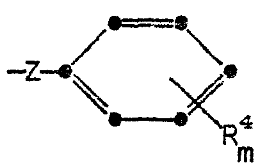

;

$R^3$ is $C_1$-$C_{10}$ alkyl, $C_3$-$C_8$ cycloalkyl, $C_3$-$C_8$ cycloalkyl bearing one or two substituents selected from the group consisting of $C_1$-$C_4$ alkyl, halogen, $C_1$-$C_4$ haloalkyl and $C_1$-$C_4$ haloalkoxy or $R^3$ is

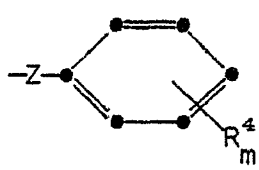

;

$R^4$ is halogen, $C_1$-$C_4$ alkyl, $C_1$-$C_4$ alkoxy, $C_1$-$C_4$ haloalkyl or $C_1$-$C_4$ haloalkoxy;

Z is $-CH_2-(CH_2)_m-O-$ or a direct link;

each m is independently 0, 1 or 2; or

$R^2$ and $R^3$, when taken together with the carbon atom to which they are attached, form dibenzo-[A,D]cyclohepta[1,4]dienylidene, 9-fluorenylidene, 9-thioxanthenylidene, 10,11-dihydro-5H-dibenzo[A,D]-cyclohepten-5-ylidene or 1,2,3,4-tetrahydronaphthalen-1-ylidene;

or an agriculturally-acceptable salt thereof.

The present invention also provides a compound of the formula (II):

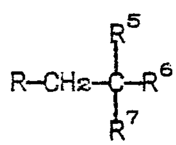

(II)

wherein:

R is 3-pyridyl, 2-pyrazinyl, 3- or 4-pyridazinyl, or 2-, 4- or 5-pyrimidinyl;

$R^5$ is halogen, hydroxy, methoxy or ethoxy;

$R^6$ is $C_3$-$C_6$ alkyl or $C_3$-$C_6$ cycloalkyl;

$R^7$ is 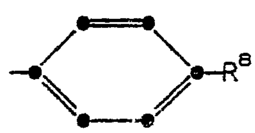 ;

$R^8$ is halogen, $C_1$-$C_4$ haloalkyl or $C_1$-$C_4$ haloalkoxy;

or an agriculturally-acceptable salt thereof.

The compounds employed in this invention are conveniently prepared by methods well known in the art. The preferred reaction method for the preparation of compounds of the invention wherein $R^1$ is hydroxy involves reacting a methyl heterocycle analog with an alkali metal derivative, followed by addition of the corresponding ketone or aldehyde to obtain the desired product.

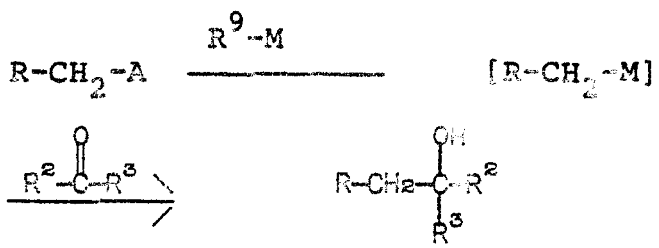


wherein R, $R^2$ and $R^3$ are as defined above and $R^9$ is $C_1$-$C_6$ alkyl, A is hydrogen or halogen. and M is an alkali metal such as lithium.

The reaction is usually carried out in a suitable solvent and at a low temperature in an inert atmosphere. Suitable solvents should be water-free and include hydrocarbons, for example toluene, hexane, benzene, and aprotic solvents such as ethers, for example diethyl ether and tetrahydrofuran, and the like. The preferred solvents include diethyl ether and tetrahydrofuran. The temperature range of the reaction mixture may be from about -10°C to -100°C with 0°C to -15°C being preferred. It is preferable to include in the mixture reactants capable of promoting the addition of the alkali metal. Examples of suitable

BAD ORIGINAL

reactants include HMPA (hexamethylphosphoramide), and hindered amines such as diisopropylamine and TMEDA (N,N,N',N'-tetramethylethylenediamine). The preferred alkali metal derivative for use in this reaction is n-butyl lithium. The reaction mixture is then worked up in accordance with usual procedures. Typically, water is added to the reaction mixture and the solids filtered off or the organic layer separated, dried and concentrated. The product may be purified by either crystallization from a suitable solvent or column chromatography according to standard procedures.

The compounds employed in the present invention wherein $R^1$ is hydroxy may also be prepared by reacting an (N-heterocyclic methyl)ketone with an appropriately substituted Grignard reagent or alkali metal derivative to provide the corresponding ethanol derivative. The reaction scheme for this process is as follows:

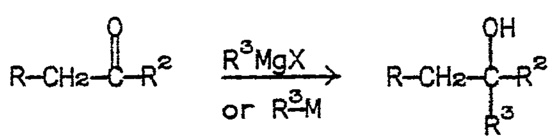


wherein R, $R^2$ and $R^3$ are as defined above and M is an alkali metal such as lithium and X is halogen. This reaction is preferred when the appropriately substituted ketone necessary in the preferred synthetic procedure, as disclosed herein, is not readily available.

The ketones and aldehydes used as starting materials in the reaction are either commercially available or prepared by procedures well known in the

art. Three of the more commonly used procedures used to prepare such starting materials, when not commercially available, are as follows: 1) a Friedel Crafts acylation wherein n is 0 or 1;

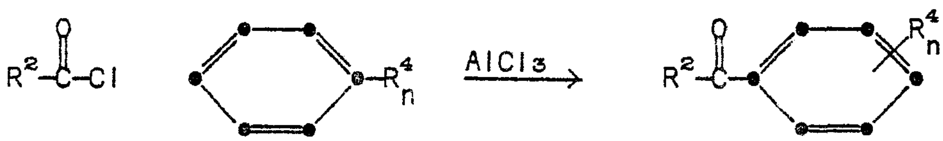


2) making the Grignard reagent of a substituted aryl halide and reacting it with the appropriately substituted acetonitrile;

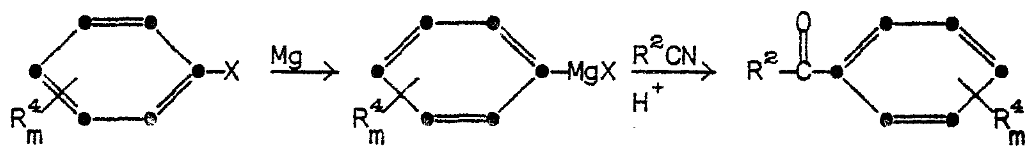


and, 3) making the Grignard reagent and reacting it with the appropriate aldehyde, followed by oxidation to the ketone;

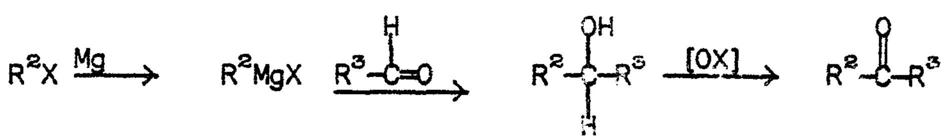


The compounds of the present invention wherein $R^1$ is methoxy or ethoxy are also prepared by procedures known in the art, or analogous to such prior art procedures. Starting with the α,α-disubstituted-α-halo-N-heterocyclic ethane derivative, which can be prepared by reacting the appropriate N-heterocyclic ethanol

derivative with a known halogenating agent such as thionyl chloride, oxalyl bromide or oxalyl chloride, and reacting it with sodium methoxide or ethoxide, as desired, the corresponding compound of the invention may be prepared. It should also be apparent that when sodium hydroxide is reacted with the α-halo derivative, the hydroxy derivative may also be prepared. This reaction is preferred when the α-halo analog is prepared from other than the corresponding ethanol derivative. The scheme for this reaction is as follows:

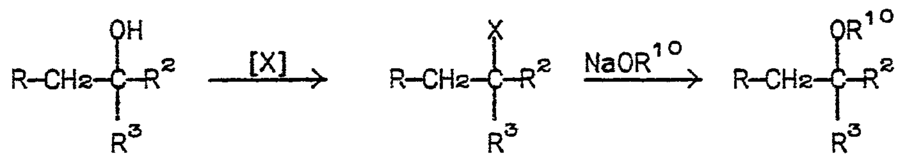


wherein X represents halogen and $R^{10}$ represents hydrogen, methyl or ethyl.

The reaction in which the halo derivative is prepared is usually carried out in a suitable solvent at an elevated temperature, usually at the reflux temperature of the reaction mixture. Suitable solvents include most hydrocarbon solvents, preferably benzene, toluene, the xylenes, chloroform, carbon tetrachloride, dichloromethane and the like. The reaction is worked up by removing the solvent <u>in vacuo</u> and purifying according to normal procedures.

The final step of the reaction outlined above is typically carried out in alcohol at a temperature in the range of from about 0°C to 100°C with 20°C to 80°C being preferred. The sodium alkoxide used is either commercially available or prepared by reacting sodium

metal with either methanol or ethanol. The reaction is worked up by removing the solvent _in vacuo_, and the resulting compound may be purified by either crystallization or column chromatography if desired.

Suitable agriculturally-acceptable salts of the compounds provided by the present invention can be prepared by methods well known in the art. Examples of such include both acid addition salts, such as hydrochloric, hydrobromic, sulfuric, phosphoric, methanesulfonic, and the like, and quaternary ammonium salts, such as methyl iodide and ethyl bromide.

Thus, the invention provides a process for preparing a compound of the formula (II):

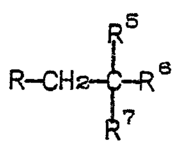 (II)

wherein:

R is 3-pyridyl, pyrazinyl, 3- or 4-pyridazinyl, or 2-, 4- or 5-pyrimidinyl;

$R^5$ is halogen, hydroxy, methoxy or ethoxy;

$R^6$ is $C_3$-$C_6$ alkyl or $C_3$-$C_6$ cycloalkyl;

$R^7$ is 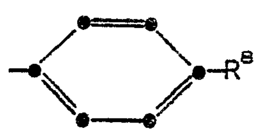 ;

$R^8$ is halogen, $C_1$-$C_4$ haloalkyl or $C_1$-$C_4$ haloalkoxy;

or an agriculturally-acceptable salt thereof;

which comprises:

(A) reacting a compound of the formula (III)

$$RCH_2M \qquad (III)$$

wherein R is as defined in formula (II) and M is an alkali metal, with a compound of formula (IV)

$$R^{6}\overset{O}{\overset{\|}{C}}R^{7} \qquad (IV)$$

wherein $R^6$ and $R^7$ are as defined in formula II, in a nonaqueous organic solvent, or

(B) reacting a compound of the formula (V)

$$R-CH_2-\overset{O}{\overset{\|}{C}}-R^{7} \qquad (V)$$

wherein R and $R^7$ are as defined in formula (II), with a compound of the formula (VI) or (VII)

$$R^{6}MgX \qquad (VI)$$

$$R^{6}M \qquad (VII)$$

wherein $R^6$ is as defined in formula II, X is halogen, and M is an alkali metal, in a nonaqueous organic solvent, or

(C) reacting a compound of the formula (VIII)

$$R-CH_2-\overset{O}{\overset{\|}{C}}R^{6} \qquad (VIII)$$

wherein R and $R^6$ are as defined in formula (II) with a compound of the formula (IX) or (X)

$R^7MgX$ (IX)

$R^7M$ (X)

wherein $R^7$ is as defined in formula (II), X is halogen, and M is an alkali metal, in a nonaqueous organic solvent, or

(D) reacting a compound of formula (II) wherein $R^5$ is hydroxy with a halogenating reagent to provide a compound of formula (II) wherein $R^5$ is halogen, or

(E) reacting a compound of formula II wherein $R^5$ is halogen with a compound of formula (XI)

$NaOR^{10}$ (XI)

wherein $R^{10}$ is hydrogen, methyl or ethyl, to provide a compound of formula (II) wherein $R^5$ is hydroxy, methoxy, or ethoxy, or

(F) salifying a compound of formula (II).

The following examples are typical of these reactions. In addition to the given physical chemistry data, each compound structure was verified by nuclear magnetic resonance (NMR). Infrared spectroscopy (IR) was performed for certain of these compounds as well.

Example 1

5-Hydroxy-5-(3-pyridylmethyl)dibenzo[A,D]-cyclohepta[1,4]diene

A mixture of 10 g. of diisopropylamine dissolved in 250 ml. tetrahydrofuran was cooled to 0°C under nitrogen. To this solution was added 45 ml. of 22% n-butyl lithium dropwise. Approximately one half hour after the addition was complete, 18 g. of HMPA was added to the reaction mixture. The mixture was cooled to approximately -70°C with a dry ice/acetone bath and 9.3 g. of 3-methylpyridine was added dropwise. Again the mixture was allowed to stir for about one half hour while maintaining the -70°C temperature, and 20.6 g. of dibenzosuberone was added. The dry ice/acetone bath was removed, and the reaction mixture was stirred overnight and allowed to come to room temperature. The mixture was poured into an ice/HCl solution and extracted with ether. The aqueous layer was made basic and again extracted with ether. The ether extracts were combined, washed with water, dried and concentrated under vacuum. The resulting oil was chromatographed over silica gel and eluted with 20% acetone/toluene (v/v). The desired fractions were combined and crystallized to afford 0.7 g. of 5-hydroxy-5-(3-pyridylmethyl)dibenzo[A,D]-cyclohepta[1,4]diene. M.P. = 145°-146°C.

Analysis calculated for $C_{21}H_{19}NO$

Theory: C, 83.69; H, 6.35; N, 4.65;

Found: C, 83.44; H, 6.36; N, 4.36.

## Example 2

α-(2-Chlorophenyl)-α-(4-chlorophenyl)-3-pyridineethanol

To a mixture of 7.1 g. diisopropylamine dissolved in 200 ml. tetrahydrofuran at 0°C under nitrogen was added 32 ml. of 22% n-butyl lithium in hexane dropwise. The reaction mixture was allowed to stir for about one half hour while maintaining the 0°C temperature and 12.6 g. of HMPA was added. The mixture was cooled to -70°C and 6.5 g. of 3-methylpyridine was added dropwise. After another 30 minutes, 17.6 g. of 2,4'-dichlorobenzophenone was added to the solution which was stirred overnight and allowed to come to room temperature. The solution was poured into 400 ml. of 10% HCl and extracted with diethyl ether. The aqueous phase was made basic with potassium hydroxide and was again extracted with ether. The ether extracts were combined, washed with water, dried, and concentrated under vacuum. The resulting residue crystallized from ether/Skellysolve B to afford 5.5 g. of α-(2-chlorophenyl)-α-(4-chlorophenyl)-3-pyridineethanol. M.P. = 173°-174°C.

Analysis calculated for $C_{19}H_{16}Cl_2NO$
Theory: C, 66.29; H, 4.39; N, 4.07;
Found: C, 66.42; H, 4.48; N, 4.23.

## Example 3

α-(1-Methylethyl)-α-(4-chlorophenyl)pyrazineethanol was prepared by reacting 12.5 g. diisopropylamine with 22.5 g. HMPA, 80 ml. of 1.6 M n-butyl lithium, 11.5 2-methylpyrazine and 22.0 g. isopropyl,

4-chlorophenyl ketone at about 0°C in 50 ml. tetrahydrofuran by the general method of Example 1. M.P. = 69°-72°C.

Analysis calculated for $C_{15}H_{17}ClN_2O$

Theory: C, 65.10; H, 6.19; N, 10.12;

Found: C, 65.06; H, 6.10; N, 10.08.

Example 4

α-(1-Methylethyl)-α-(4-chlorophenyl)-5-pyrimidineethanol was prepared by reacting 12.5 g. diisopropylamine with 22.5 g. HMPA, 80 ml. of 1.6 M n-butyl lithium, 12 g. of 5-methylpyrimidine and about 22 g. of isopropyl, 4-chlorophenyl ketone at about 0°C in 100 ml. tetrahydrofuran by the general procedure of Example 1. Yield 1.2 g. M.P. = 156°C.

Analysis calculated for $C_{15}H_{17}ClN_2O$

Theory: C, 65.10; H, 6.19; N, 10.12;

Found: C, 65.19; H, 6.10; N, 9.98.

By following the general procedures outlined above, additional compounds were prepared.

Example 5

α-(2-Chlorophenyl)-α-(4-chlorophenyl)pyrazineethanol

M.P. = 119°C.

Analysis calculated for $C_{18}H_{14}Cl_2N_2O$

Theory: C, 62.62; H, 4.09; N, 8.11;

Found: C, 62.65; H, 4.25; N, 7.73.

Example 6

α-(1-Methylethyl)-α-(4-chlorophenyl)-3-pyridineethanol

M.P. = 109°-110°C.

Analysis calculated for $C_{16}H_{18}ClNO$

Theory: C, 69.68; H, 6.58; N, 5.08;

Found: C, 69.48; H, 6.37; N, 5.03.

Example 7

α,α-Dihexyl-3-pyridineethanol

oil

NMR: Multiplet from δ 0.9 to 1.5 (27 protons); singlet at δ 2.7 (2 protons); multiplet at δ 7.1 to δ 7.7 (2 protons); and singlet at δ 8.4 (2 protons).

Example 8

α-(1,1-Dimethylethyl)-α-(4-methoxyphenyl)-3-pyridineethanol

M.P. = 125°-126°C.

Analysis calculated for $C_{18}H_{23}NO_2$

Theory: C, 75.76; H, 8.12; N, 4.91;

Found: C, 75.96; H, 7.92; N, 4.81.

Example 9

α-Cyclohexyl-α-(2,5-dimethylcyclohexyl)-3-pyridineethanol

M.P. = 105°-106°C.

Analysis calculated for $C_{21}H_{33}NO$

Theory: C, 79.95; H, 10.54; N, 4.44;

Found: C, 79.71; H, 10.34; N, 4.43.

Example 10

α-(1-Methylethyl)-α-[4-(trifluoromethyl)-phenyl]pyrazineethanol

oil

Analysis calculated for $C_{16}H_{17}F_3N_2O$

Theory: C, 61.93; H, 5.52; N, 9.03;

Found: C, 61.72; H, 5.37; N, 8.98.

Example 11

α-Cyclopropyl-α-(4-chlorophenyl)pyrazine-ethanol

oil

NMR: triplet at δ 0.4 (4 protons); multiplet at δ 1.2 (1 proton); singlet at δ 3.4 (2 protons); singlet at δ 5.2 (1 proton); multiplet at δ 7.3 (4 protons); and singlet at δ 8.4 (3 protons).

Example 12

9-(3-Pyridylmethyl)-9H-fluoren-9-ol

M.P. = 108°-109°C.

Analysis calculated for $C_{17}H_{15}NO$

Theory: C, 83.49; H, 5.53; N, 5.12;

Found: C, 83.25; H, 5.45; N, 5.32.

Example 13

α-(1-Methylethyl)-α-(4-methoxyphenyl)-2-pyridineethanol

M.P. = 55°-56°C.

Analysis calculated for $C_{17}H_{21}NO_2$

Theory: C, 75.25; H, 7.80; N, 5.16;

Found: C, 75.01; H, 7.59; N, 4.91.

Example 14

α-(1,1-Dimethylethyl)-α-(4-methoxyphenyl)-5-pyrimidineethanol

M.P. = 152°-153°C.

Analysis calculated for $C_{17}H_{22}N_2O_2$

Theory: C, 71.30; H, 7.74; N, 9.78;

Found: C, 71.50; H, 7.52; N, 9.82.

Example 15

α-(1,1-Dimethylethyl)-α-(4-fluorophenyl)-pyrazineethanol

M.P. = 80°-81°C.

Analysis calculated for $C_{16}H_{19}FN_2O$

Theory: C, 70.07; H, 6.93; N, 10.22;

Found: C, 70.46; H, 6.85; N, 10.49.

Example 16

α-Cyclohexyl-α-[4-(1,1,2,2-tetrafluoroethoxy)-phenyl]pyrazineethanol

M.P. = 62°-63°C.

Analysis calculated for $C_{20}H_{22}F_4N_2O_2$

Theory: C, 60.30; H, 5.57; N, 7.03;

Found: C, 60.16; H, 5.41; N, 7.02.

Example 17

α-(1-Methylethyl)-α-[4-(pentafluoroethoxy)-phenyl]pyrazineethanol

oil

Analysis calculated for $C_{17}H_{17}F_5N_2O_2$

Theory: C, 54.26; H, 4.55; N, 7.44;

Found: C, 54.50; H, 4.26; N, 7.29.

Example 18

α-(1,1-Dimethylethyl)-α-(4-methoxyphenyl)-pyrazineethanol

M.P. = 104°-105°C.

Analysis calculated for $C_{17}H_{22}N_2O_2$

Theory: C, 71.30; H, 7.74; N, 9.78;

Found: C, 71.53; H, 7.62; N, 10.04.

Example 19

9-(Pyrazinylmethyl)-9H-thioxanthen-9-ol

M.P. = 147°-148°C.

Analysis calculated for $C_{19}H_{16}N_2OS$

Theory: C, 70.56; H, 4.61; N, 9.14;

Found: C, 70.76; H, 4.69; N, 9.38.

Example 20

10,11-Dihydro-5-(pyrazinylmethyl)-5H-dibenzo-[A,B]cycloheptan-5-ol

M.P. = 101°-102°C.

Analysis calculated for $C_{20}H_{18}N_2O$

Theory: C, 79.44; H, 6.00; N, 9.26;

Found: C, 79.64; H, 5.74; N, 9.03.

Example 21

1,2,3,4-Tetrahydro-1-(pyrazinylmethyl)-1-naphthalenol

M.P. = 88°C.

Analysis calculated for $C_{15}H_{16}N_2O$

Theory: C, 74.97; H, 6.71; N, 11.66;

Found: C, 74.79; H, 6.60; N, 11.44.

## Example 22

α-(1-Methylethyl)-α-[4-(trifluoromethoxy)-phenyl]pyrazineethanol

oil

Analysis calculated for $C_{16}H_{17}F_3N_2O_2$

Theory: C, 58.89; H, 5.25; N, 8.58;

Found: C, 58.64; H, 5.46; N, 8.34.

## Example 23

α-(1-Methylethyl)-α-[4-(trifluoromethoxy)-phenyl]-3-pyridineethanol

M.P. = 118°C.

Analysis calculated for $C_{17}H_{18}F_3NO_2$

Theory: C, 62.76; H, 5.58; N, 4.31;

Found: C, 62.57; H, 5.74; N, 4.25.

## Example 24

α-(1-Methylethyl)-α-[4-(trifluoromethoxy)-phenyl]-5-pyrimidineethanol

M.P. = 146°-147°C.

Analysis calculated for $C_{16}H_{17}F_3N_2O_2$

Theory; C, 58.89; H, 5.25; N, 8.58;

Found: C, 58.77; H, 5.48; N, 8.39.

## Example 25

α-(n-Hexyl)-α-(2-chlorophenyl)pyrazineethanol

oil

Analysis calculated for $C_{18}H_{23}ClN_2O$

Theory: C, 67.81; H, 7.27; N, 8.79;

Found: C, 68.03; H, 7.03; N, 8.57.

Example 26

α-(1,1-Dimethylethyl)-α-[(4-chlorophenoxy)-methyl]pyrazineethanol

M.P. = 70°-71°C

Analysis calculated for $C_{17}H_{21}ClN_2O_2$

Theory: C, 63.65; H, 6.60; N, 8.73;

Found: C, 63.78; H, 6.65; N, 8.57.

Example 27

α-(1,1-Dimethylethyl)-α-[(4-chlorophenoxy)-methyl]-3-pyridineethanol hydrochloride

M.P. = 213°-215°C

Analysis calculated for $C_{18}H_{23}Cl_2NO_2$

Theory: C, 60.58; H, 6.51; N, 3.93;

Found: C, 60.76; H, 6.39; N, 3.97.

Example 28

α-Phenyl-α-(2,4-dichlorophenyl)pyrazineethanol

M.P. = 142°-144°C

Analysis calculated for $C_{18}H_{14}Cl_2N_2O$

Theory: C, 62.62; H, 4.09; N, 8.11;

Found: C, 62.84; H, 4.29; N, 7.90.

Example 29

α-Cyclobutyl-α-(4-fluorophenyl)pyrazineethanol

oil

Analysis calculated for $C_{16}H_{17}FN_2O$

Theory: C, 70.57; H, 6.29; N, 10.29;

Found: C, 70.34; H, 6.02; N, 10.46.

The present invention provides a method for controlling the growth of aquatic plants which comprises adding to the water containing said plants a growth-regulating and non-herbicidal amount of a present ethanol derivative.

The concentration of compound present in the body of water to be treated depends on the effect desired and plant species treated. When the compounds employed in the present invention are added to the water containing submerged and floating aquatic plants for which control is desired, a concentration range of from about 0.10 to about 20 ppm. of the compound is desired. It is, of course, apparent that higher or lower concentrations can be employed depending on the plant species to be inhibited, the temperature, and the shape and type of the body of water to be treated. At higher water temperatures, for example, less compound is generally required for a given degree of control than is needed at lower temperatures.

In considering the treatment of moving streams for the purpose of controlling flora fixed therein, special account must be taken of the fact that the compounds will pass over the area to be treated and that the concentration during the contact period is dependent upon the water flow rate, the rate of chemical addition, and the time period of addition.

Since the compounds provided herein appear to function most effectively when completely soluble in the water which contains the plants to be controlled, a preferred aspect of this invention is that the compounds be in the form of an agriculturally-acceptable salt when used aquatically. In general, however, the substituted free base compounds employed in the present invention are as effective as their corresponding salts in controlling aquatic plant growth in a body of water when appropriately formulated.

The aquatic plant growth regulator activity of representative compounds of the present invention is illustrated by the following experiments.

## Experiment 1

The initial screening procedure used to detect compounds which show aquatic plant growth regulator activity was conducted at a concentration of 10 ppm. Test compounds were formulated by adding 1.0 ml. acetone and 9.0 ml. of an aqueous 0.1 percent Tween 80 solution (polyoxyethylene (20) sorbitan mono-oleate) to 20 mg. test compound. To obtain 10 ppm., 4.00 ml. of this solution, designated stock solution A, was added to 785 ml. water fortified with 3.0 ml. of Hoagland's Nutrient solution. To the container holding the above solutions were added three 10 cm. terminal, unbranched cuttings of *Hydrilla verticillata* (herein called hydrilla). Two containers are prepared for each compound to be tested. Controls were also run in separate containers which include the same components as the test containers, excluding the test compound.

Measurements were made of the total plant growth of each plant after a period of two to three weeks. An average total growth was obtained by dividing the total combined lengths of hydrilla by the number of replicates. The average increase in growth was obtained by subtracting the original hydrilla length of 10 cm. This difference was divided by the average increase in growth of controls and this quotient multiplied by 100 to give a percent inhibition.

$$\frac{\text{Total combined length of Replicates}}{\text{Number of Replicates}} = \text{Average Length}$$

$$\text{Average Length} - 10\text{ cm} = \text{Average Increased Growth}$$

$$\left[1 - \frac{\text{Average Increased Growth}}{\text{Average Increased Growth of Control}}\right] \times 100 = \%\text{ inhibition}$$

The results of the test are set forth in Table 1 which follows. In Table 1, the number in column 1 identifies the test compound; column 2 lists the percent growth inhibition of hydrilla observed. One of the compounds was tested more than once at this concentration.

Table 1

| Example No. of Compound Tested | Percent Growth Inhibition at 10 ppm. |
|---|---|
| 1 | 52 |
| 2 | 48, 70 |
| 3 | 99 |
| 4 | 96 |
| 5 | 79 |
| 6 | 99 |
| 7 | 100 |
| 8 | 63 |
| 9 | 71 |
| 12 | 95 |
| 13 | 29 |
| 14 | 59 |
| 15 | 94 |
| 16 | 79 |
| 17 | 85 |
| 18 | 32 |
| 19 | 68 |
| 20 | 66 |
| 21 | 18 |
| 22 | 93 |
| 23 | 91 |
| 24 | 81 |

Experiment 2

Test compounds were further evaluated at concentrations of 1.0, 0.5, and 0.25 ppm. according to the general procedure outlined above. Formulation of a test compound concentration of 1.0 ppm. was done by combining 4 ml. of stock solution A, as prepared in Experiment 1, with 36 ml. 0.1 percent Tween 80 to give stock solution B. Four ml. of stock solution B was added to 785 ml. water and 3 ml. Hoagland's nutrient in a container as above.

The 0.5 ppm. test concentration was obtained by adding 20 ml. of Stock B to 20 ml. 0.1 percent Tween 80 to give stock solution C. Four ml. of stock solution C was combined with 785 ml. $H_2O$ and 3 ml. Hoagland's nutrient in a container with hydrilla to give test results at 0.5 ppm.

The 0.25 ppm. test concentraton was obtained by combining 20 ml. of stock C with 20 ml. 0.1 percent Tween 80 to give stock solution D. Four ml. of stock solution D was combined with 785 ml. $H_2O$ and 3 ml. Hoagland's nutrient in a container with hydrilla.

Observations were made in the same manner as described above, and the percent inhibition observed was calculated using the formulas set forth above as well. Again, some compounds were tested more than once at a specific concentration. This data is summarized in Table 2.

Table 2

| Example No. of Compound Tested | Percent Growth Inhibition at Indicated Test Concentrations 1 ppm. | 0.5 ppm. | 0.25 ppm. |
|---|---|---|---|
| 1 | 16 | 7 | 15 |
| 2 | 63,33 | 26,56 | 26,61 |
| 3 | 58,78 | 23,40 | -27,-6 |
| 4 | 48,48,94,51 | 25,13,13,29 | 34,1,31,9 |
| 5 | 2 | 9 | 9 |
| 6 | 19,71,51 | 26,57,34 | 25,36,13 |
| 8 | 50 | 6 | 10 |
| 9 | 24 | 31 | 4 |
| 12 | 12 | -2 | 5 |
| 15 | -1 | 12 | 12 |
| 16 | 6 | 13 | 16 |
| 17 | -1 | 35 | 36 |
| 19 | 44,13 | 37,-2 | 29,2 |
| 20 | 43,20 | 30,0 | 4,15 |
| 22 | 42 | 22 | 26 |
| 23 | 67 | 51 | 30 |

The present invention also provides a method for controlling the growth of terrestrial plants which comprises applying to the plants or the locus of the plants for which control is desired a growth-regulating and non-herbicidal amount of a present active agent.

The term "growth-regulating and non-herbicidal amount", as defined herein, refers to an amount of a present ethanol derivative which either reduces or regulates the growth of the plant species for which control is desired. This amount will generally be from about 1 ppm to 2000 ppm, more preferably from about 50 ppm to 1000 ppm of a present active agent. The exact concentration of active ingredient required varies with the plant species to be controlled, type of formulation, soil type, climate conditions and the like.

The present active agents may be applied to the soil surrounding emerged plants or to the plants themselves where plant growth regulator activity is sought.

The terrestrial plant growth regulator activity of representative compounds employed in the present invention is illustrated by the following experiment.

Experiment 3

The initial screen used to detect terrestrial plant growth regulator activity was conducted with squash, snap beans, barley and soybeans. The test compounds were formulated for application by dissolving the compound into a solvent prepared by combining Toximul R and Toximul S (proprietary blends of anionic and nonionic surfactants manufactured by Stepan Chemical Company, Northfield, Illinois) in deionized water to give a final concentration of 1000 ppm. The formu-

lation was sprayed onto the test plants to the drip point. The plants were allowed to dry and were placed in the greenhouse. After 5 days the plants were evaluated for growth retardation or enhancement and chemical injury. The following rating system was used:

+ = promotion
- = inhibition
0 = no effect
1 = slight effect
2 = moderate effect
3 = distinct effect

The following letter descriptions for chemical injury were also used:

B = burn
G = darker green
C = chlorosis
M = morphological changes
X = death
P = pinching of the terminal meristem

The results of this experiment appear below in Table 3.

Table 3

Terrestrial Plant Growth Regulation

| Example No. of Compound Tested | Squash | | Bean | | | | Barley | Soybean |
|---|---|---|---|---|---|---|---|---|
| | Growth Rating | Phyto-toxicity | Growth Rating | Abscission Rating | Morpho-logical Effects | Phyto-toxicity | Growth and Phyto-toxicity Rating | Growth and Phyto-toxicity Rating |
| 1 | | | -1 | 0 | 0 | | -1B | -3B |
| 2 | 0 | 0 | 0 | 0 | 0 | 0 | | |
| 3 | -3 | 3X | -3 | 0 | 2 | 0 | | |
| 4 | -2 | 0 | -1 | 0 | 1 | 0 | | |
| 5 | 0 | 0 | -3 | 0 | 0 | 0 | | |
| 6 | -1 | 0 | -1 | 0 | 3 | 1C | | |
| 7 | -3 | 3B | 2 | 0 | 2 | 0 | | |
| 8 | -3 | 0 | 0 | 0 | 1 | 0 | | |
| 9 | | | -3 | 1 | 3C | | 0 | 0 |
| 11 | 0 | 0 | -3 | 0 | 0 | 2B | | |
| 12 | 0 | 0 | 0 | 1 | 0 | 0 | | |
| 15 | | | -1 | 0 | 0 | 0 | 0 | 0 |
| 16 | | | -2 | 0 | 0 | | 0 | 0 |
| 17 | | | 0 | 1 | 0 | | -2X | -1M |
| 18 | | | -1 | 0 | 0 | | -1 | 0M |
| 19 | | | 0 | 1 | 0 | | 2 | 0C |
| 20 | | | 0 | 0 | 0 | | 0 | 0 |
| 21 | | | -3 | 3 | 3 | | 0 | 0 |

The present invention also provides a method for controlling the growth of fungal diseases which comprises applying to a locus of a plant a disease inhibiting and non-herbicidal amount of a present active agent. The term "disease inhibiting and non-herbicidal amount," as used herein, refers to an amount of a compound of the invention which either kills or stunts the fungal disease for which control is desired, but is not significantly toxic to the plant. This amount will generally be from about 1 to 1000 ppm, with 10 to 500 ppm being preferred. The exact concentration of compound required varies with the fungal disease to be controlled, the type formulation employed, the particular plant species, climate conditions and the like.

The fungicidal efficacy of representative compounds of the present invention is illustrated by the following experiments.

Experiment 4

This initial screen was used to evaluate the efficacy of the compounds employed in the present invention against a variety of different agents causing fungal diseases.

The test compounds were formulated for application by dissolving 70 mg. of the compound into 2 ml. of solvent. The solvent was prepared by mixing 100 ml. of Tween 80 with 500 ml. of acetone and 500 ml. of ethanol. The solvent/compound solution was diluted to 175 ml. with deionized water containing Dow Corning Antifoam C Emulsion (one drop per 2 liters).

The formulated test compounds were applied by both soil drench and foliar methods. In the foliar spray application method the following plant pathogens and host plants were employed.

| | |
|---|---|
| Powdery Mildew | - Bean |
| Anthracnose | - Cucumber |
| Rice Blast | - Rice |
| Botrytis | - Grape |
| Helminthosporium | - Wheat |
| Leaf Rust | - Wheat |

The foliar application was run at a test compound concentration of 400 ppm by either of two different procedures. The Botrytis test was sprayed with a small DeVilbiss atomizer at approximately 8 psi. For the remaining applications test solutions were sprayed by hand in an exhaust ventilated chamber. Single pots of different plant species were placed on raised, revolving pedestals in the chamber. Using a DeVilbiss spray gun, Model TGA-502, all test solutions were applied by hand at 40 psi. As the spray was delivered, the pedestals were rotated to expose all plant surfaces to the spray pattern. The spray was applied to past the run-off point. All treatments were allowed to dry and the host plants were inoculated with the pathogens approximately 24 hours later.

In the soil drench application method the following plant pathogen and host plant were employed.

| | |
|---|---|
| Rhizoctonia Damping-Off | - Cotton |

The soil drench method was performed by uniformly syringing 20 ml. of the formulation over the soil surface of each pot containing a different crop species. The pot size at the soil surface was 2.0 inches in diameter, thus equalling a test compound concentration of 35 lbs./acre.

The effectiveness of the test compounds in controlling the foregoing plant diseases was rated on a scale of 1 to 5. On this scale "1" indicates severe disease (or no control), "2" is moderate disease, "3" is slight disease, "4" is very slight disease and "5" indicates no disease or 100% control. Also a phytotoxicity rating was recorded again using a scale from 1 to 5 where 1 indicates no toxicity and 5 indicates death to the plant. Where no rating is given in the tables which follow, it is to be presumed that a 1 is appropriate, i.e., no phytotoxicity was present. Finally, where phytotoxicity was present, a letter rating may be given to the plant indicating the type of injury caused to the plant. These injuries were coded as follows:

G = General necrosis
W = Wilting
S = Stunting
C = Chlorosis
F = Formative

Table 4 presents the activity of typical ethanol derivatives of the present invention when evaluated in the foliar application method described above, while Table 5 presents the results of the soil drench application method. Where plant injury occurred, the rating is given in parenthesis.

Table 4

Foliar Spray at 400 ppm

Disease Control (Phytotoxicity Rating)

| Example No. of Compound Tested | Powdery Mildew | Anthracnose | Rice Blast | Botrytis | Helminthosporium | Leaf Rust |
|---|---|---|---|---|---|---|
| 1 | 3 | 1 | 3 | 1 | 1 | 1 |
| 2 | 1 | 1 | 1 | 1 | 1 | 1 |
| 3 | 5(2C) | 1 | 1 | 1 | 4 | 1 |
| 4 | 5 | 1 | 1 | 1 | 1 | 1 |
| 5 | 1 | 1 | 1 | 1 | 1 | 1 |
| 6 | 4 | 1 | 1 | 1 | 4 | 1 |
| 7 | 4 | 1 | 1 | 1 | 1 | 1 |
| 8 | 1 | 1 | 1 | 1 | 1 | 1 |
| 9 | 1 | 1 | 1 | 1 | 1 | 1 |
| 10 | 5 | 1 | 1 | 1 | 1 | 1(2S) |
| 11 | 1 | 1 | 3 | 1 | 4 | 1 |
| 12 | 1 | 1 | 1 | 1 | 1 | 1 |
| 15 | 4 | 1 | 1 | 1 | 3 | 1 |
| 16 | 5 | 1 | 1 | 1 | 1 | 4(2C) |
| 17 | 4 | 1 | 1 | 1 | 1 | 1 |
| 18 | 5 | 1 | 1 | 1 | 1 | 1 |
| 19 | 1 | 1 | 4 | 1 | 1 | 1 |
| 20 | 1 | 1 | 1 | 1 | 1 | 1 |
| 21 | 1 | 1 | 1 | 1 | 1 | 1 |
| 22 | 5 | 1 | 1 | 1 | 1 | 1 |
| 23 | 5 | 1 | 1 | 1 | 1 | 1 |
| 24 | 5 | 1 | 1 | 1 | 1 | 1 |
| 25 | 4 | 1(4G) | 1 | 1 | 1 | 1 |

Table 5

Soil Drench at 35 lbs./acre
(39.2 kg./ha.)

Disease Control (Phytotoxicity Rating)

| Example No. of Compound Tested | Rhizoctonia Damping-Off |
|---|---|
| 1 | 1 |
| 2 | 4 |
| 3 | 4(4S) |
| 4 | 1 |
| 5 | 1 |
| 7 | 3 |
| 8 | 1 |
| 9 | 3 |
| 10 | 3 |
| 11 | 5(4S) |
| 12 | 1 |
| 15 | 3 |
| 16 | 1 |
| 17 | 1 |
| 18 | 1 |
| 19 | 1 |
| 20 | 1 |
| 21 | 3 |
| 22 | 3 |
| 23 | 1 |
| 24 | 1 |
| 25 | 3 |

Some of the compounds tested in the initial screen outlined above were evaluated in one or more of the following secondary screens.

Experiment 5

Compounds tested in this plant disease foliage screen were formulated in the same manner as described above for Experiment 4. The 400 ppm concentration obtained by this procedure was then serially diluted to obtain solutions having a lower concentration of test compound. The formulations were sprayed on the plants in the same manner as described above. One day after treatment, the host plants were inoculated with the pathogen spores. The diseases and their corresponding host plants used in this experiment are as follows:

| | | |
|---|---|---|
| Powdery mildew | - | Bean |
| Late blight | - | Tomato |
| Apple Scab | - | Apple |
| Anthracnose | - | Cucumber |
| Rice blast | - | Rice |
| Downy mildew | - | Grape |
| Cercospora leafspot | - | Sugar beet |

After a suitable incubation period, when disease symptoms appeared on untreated control plants, treatments were rated for disease severity according to the rating system described above. The results are recorded in Table 6.

Table 6

| Example No. of Compound Tested | Application Rate (ppm.) | Powdery Mildew | Late Blight | Apple Scab | Anthracnose | Rice Blast | Downy Mildew | Cercospora Leaf Spot |
|---|---|---|---|---|---|---|---|---|
| 4 | 400 | 4(2G) | 1 | 1 | 4(2G) | 1 | 1(2G) | 3(2G) |
| | 400 | 5(2G) | | | 4(2G) | | | |
| | 100 | 1 | | | 1 | | | |
| | 25 | 1 | | | 1 | | | |
| 10 | 400 | 5 | 1(3G) | 5(2G) | 1 | 1 | 3 | 1 |
| | 400 | 5 | 1 | 5 | 1 | 1 | 3 | 1 |
| | 400 | 5 | | 5(2G) | | | | |
| | 100 | 5 | | 4 | | | | |
| | 25 | 4 | | 1 | | | | |
| | 6 | 3 | | 1 | | | | |
| 15 | 400 | 4 | 1 | 5 | 1 | 1 | 1 | 1 |
| | 400 | 4 | | 5 | | | | |
| | 100 | 1 | | 1 | | | | |
| | 25 | 1 | | 1 | | | | |
| 16 | 400 | 5 | 1 | 5 | 1 | 3 | 5 | 4 |
| | 400 | 5 | | 1 | | | 1 | 1 |
| | 100 | 5 | | 1 | | | 1 | 1 |
| | 25 | 1 | | 1 | | | 1 | 1 |
| 17 | 400 | 5 | 1 | 1 | 1 | 1 | 1 | 4 |
| | 400 | 5 | | | | | | 1 |
| | 100 | 4 | | | | | | 1 |
| | 25 | 1 | | | | | | 1 |
| 18 | 400 | 5 | 1 | 5 | 1 | 1 | 4 | 4 |
| | 400 | 4 | | 4 | | | | 1 |
| | 100 | 1 | | 3 | | | | 3 |
| | 25 | 1 | | 1 | | | | 1 |
| 19 | 400 | 1 | 1 | 1 | 1 | 3 | 1 | 1 |

Table 6 cont'd.

| Example No. of Compound Tested | Application Rate (ppm.) | Powdery Mildew | Late Blight | Apple Scab | Anthracnose | Rice Blast | Downy Mildew | Cercospora Leaf Spot |
|---|---|---|---|---|---|---|---|---|
| 23 | 400 | 5(2C) | 3 | 1 | 1(3C) | 1 | 4 | 1 |
| | 400 | 5 | | | | | 4 | |
| | 100 | 4 | | | | | 4 | |
| | 100 | 4 | | | | | - | |
| | 25 | 3 | | | | | 1 | |
| | 25 | 3 | | | | | | |
| | 6 | 1 | | | | | | |
| 24 | 400 | 5 | 4 | 1 | 1 | 1 | 3(2C) | 1 |
| | 400 | 5 | 4 | | | | | |
| | 100 | 4 | 3 | | | | | |
| | 25 | 1 | 3 | | | | | |
| 25 | 400 | 4 | 4 | 1 | 1 | 1 | 4 | 1 |
| | 400 | 4 | 3 | | | | 4 | |
| | 100 | 1 | 3 | | | | 1 | |
| | 25 | 1 | 1 | | | | 1 | |

Experiment 6

Certain of the compounds were tested in an effort to evaluate their fungicidal efficacy and systemic control of various cereal grain diseases. The compounds tested were formulated as above and applied both foliar spray and soil drench. The diseases and host plants employed in this test were as follows.

| | | |
|---|---|---|
| Powdery mildew | - | Wheat |
| Leaf rust | - | Wheat |
| Helminthosporium leaf spot | - | Wheat |
| Septoria leaf blotch | - | Wheat |

After a suitable incubation period when disease symptoms had appeared on untreated plants, treatments were rated for disease severity. The compounds were rated as above, and the results of the foliar test appear in Table 7, while the results of the soil drench test are presented in Table 8.

Table 7

Foliar Application

| Example No. of Compound Tested | Application Rate (ppm) | Powdery Mildew | Leaf Rust | Helminthosporium Leaf Spot | Septoria Leaf Blotch |
|---|---|---|---|---|---|
| 3 | 400 | 4 | 3 | 4 | 5 |
| | 400 | 4 | | 4 | 4 |
| | 100 | 3 | | 1 | 5 |
| | 25 | 1 | | 1 | 3 |
| 6 | 400 | 4(3G) | 4 | 1 | 5(3G) |
| | 400 | 4 | 1 | | 4(3G) |
| | 100 | 1 | | | 5(3G) |
| | 25 | 1 | | | 5(3G) |
| 9 | 400 | 1 | 1 | 1 | 3 |
| 10 | 400 | 3(2S) | 1 | 1 | 5(2S) |
| | 400 | 4(3S) | 1 | 1 | 5 |
| | 400 | 3(2S) | | | 4(2S) |
| | 400 | 4(2S) | | | 4(2S) |
| | 100 | 1(2S) | | | 4 |
| | 100 | 1 | | | 1 |
| | 25 | 1 | | | 1 |
| | 25 | 1 | | | |
| 14 | 400 | 1 | 1 | 1 | 1 |

Table 7 cont'd.

Foliar Application

| Example No. of Compound Tested | Application Rate (ppm) | Powdery Mildew | Leaf Rust | Helminthosporium Leaf Spot | Septoria Leaf Blotch |
|---|---|---|---|---|---|
| 15 | 400 | 3 | 1 | 3 | 1 |
| | 400 | 3 | | | |
| | 100 | 1 | | | |
| | 25 | 1 | | | |
| 16 | 400 | 5 | 1 | 1 | 1 |
| | 400 | 4 | | | |
| | 100 | 5 | | | |
| | 100 | 4 | | | |
| | 25 | 3 | | | |
| | 25 | 3 | | | |
| | 6 | 3 | | | |
| 17 | 400 | 5 | 1 | 1 | 4 |
| | 400 | 5 | | | 1 |
| | 100 | 5 | | | 1 |
| | 100 | 4 | | | - |
| | 25 | 4 | | | 1 |
| | 25 | 3 | | | |
| | 6 | 1 | | | |
| 18 | 400 | 5 | 1 | 1 | 4 |
| | 400 | 3 | | | 1 |
| | 100 | 3 | | | 1 |
| | 25 | 1 | | | 1 |

Table 7 cont'd.

Foliar Application

| Example No. of Compound Tested | Application Rate (ppm) | Powdery Mildew | Leaf Rust | Helminthosporium Leaf Spot | Septoria Leaf Blotch |
|---|---|---|---|---|---|
| 21 | 400 | 5 | 1(2G) | 1(2G) | 1 |
| | 400 | 3 | | | |
| | 100 | 1 | | | |
| | 25 | 1 | | | |
| 22 | 400 | 5(2S) | 3(2S) | 4(3S) | 5(3S) |
| | 400 | 4(2S) | | 4(3S) | 4(2S) |
| | 100 | 4 | | 1(2S) | 1 |
| | 25 | 3 | | 1 | 1 |
| 23 | 400 | 4(2G) | 1 | 4 | 1 |
| | 400 | 1 | | 1 | |
| | 100 | 1 | | 1 | |
| | 25 | 1 | | 1 | |
| 24 | 400 | 4(2G) | 4(2S) | 1 | 4 |
| | 400 | 5(2G) | 1 | | 3 |
| | 100 | 4 | 1 | | 1 |
| | 100 | 5(2G) | 1 | | |
| | 25 | 4 | | | 1 |
| | 25 | 1 | | | |
| | 6 | 1 | | | |
| 25 | 400 | 4 | 1 | 1 | 1 |

Table 7 cont'd.

Foliar Application

| Example No. of Compound Tested | Application Rate (ppm) | Powdery Mildew | Leaf Rust | Helminthosporium Leaf Spot | Septoria Leaf Blotch |
|---|---|---|---|---|---|
| 26 | 400 | 5 | 1 | 5 | 5(2C) |
| | 400 | 5 | | 5 | 4(2CG) |
| | 100 | 4 | | 4 | 4(2C) |
| | 100 | 1 | | 4 | 4 |
| | 25 | 4 | | 1 | 4 |
| | 25 | 1 | | 1 | 1 |
| | 6 | 1 | | 1 | 1 |
| 28 | 400 | 4 | 1 | 1 | 4 |
| | 400 | 1 | | | 4 |
| | 100 | 1 | | | 1 |
| | 25 | 1 | | | 1 |
| 29 | 400 | 5 | 1 | 5 | 5 |
| | 400 | 1(3G) | | 5 | 5 |
| | 100 | 1(2C) | | 3 | 3 |
| | 25 | 1 | | 1 | 1 |

Table 8

Soil Application

| Example No. of Compound Tested | Application Rate (lbs./acre) | Powdery Mildew | Leaf Rust | Helminthosporium Leaf Spot | Septoria Leaf Blotch |
|---|---|---|---|---|---|
| 3 | 11.0 | 5(3G) | 4(2S) | 4(25) | 4(2S) |
| | 11.0 | 4(2S) | 1(2S) | 1(2S) | 4(2S) |
| | 3.0 | 5(2G) | 1(2G) | 4(2S) | 3(2G) |
| | 0.7 | 5(2G) | 1(2G) | 3 | 3(2G) |
| 6 | 11.0 | 4(2G) | 1 | 1 | 4(3G) |
| | 11.0 | 1 | | | 1(2G) |
| | 3.0 | 1 | | | 1(2G) |
| | 0.7 | 1 | | | 1(2G) |
| 9 | 11.0 | 1 | 1 | 1 | 1 |
| 10 | 11.0 | 5(3S) | 1(2S) | 1 | 4(3S) |
| | 11.0 | 5(3S) | 1 | 1 | 4(3S) |
| | 11.0 | 5(3S) | | | 1(3S) |
| | 11.0 | 3(2S) | | | |
| | 3.0 | 5(2S) | | | 4(3S) |
| | 3.0 | 4(3S) | | | |
| | 0.7 | 1(2S) | 3(1) | | 1(2S) |
| 14 | 11.0 | 1 | 1 | 1 | 1 |
| 15 | 11.0 | 5(2S) | 1 | 1 | 1(2S) |
| | 11.0 | 4(2S) | | | |
| | 3.0 | 4 | | | |
| | 0.7 | 1 | | | |

Table 8 cont'd.

| Example No. of Compound Tested | Application Rate (lbs./acre) | Powdery Mildew | Leaf Rust | Helminthosporium Leaf Spot | Septoria Leaf Blotch |
|---|---|---|---|---|---|
| 16 | 11.0 | 3 | 1 | 4 | 1 |
| | 11.0 | 3 | | 1 | |
| | 3.0 | 1 | | 1 | |
| | 0.7 | 1 | | 1 | |
| 17 | 11.0 | 5 | 1 | 4 | 1 |
| | 11.0 | 3 | | 1 | |
| | 3.0 | 1 | | 1 | |
| | 0.7 | 1 | | 1 | |
| 18 | 11.0 | 5 | 1 | 1 | 1 |
| | 11.0 | 3 | | | |
| | 3.0 | 1 | | | |
| | 0.7 | 1 | | | |
| 21 | 11.0 | 5 | 1 | 1 | 1 |
| | 11.0 | 3 | | | |
| | 3.0 | 1 | | | |
| | 0.7 | 1 | | | |
| 22 | 11.0 | 5(3S) | 3(2S) | 1(3S) | 4(3S) |
| | 11.0 | 5(3S) | | | 5(3S) |
| | 3.0 | 5(2S) | | | 5(2S) |
| | 0.7 | 5 | | | 1 |
| 23 | 11.0 | 4 | 1 | 1 | 1 |
| | 11.0 | 1 | | | |
| | 3.0 | 1 | | | |
| | 0.7 | 1 | | | |

Table 8 cont'd.

| Example No. of Compound Tested | Application Rate (lbs./acre) | Powdery Mildew | Leaf Rust | Helminthosporium Leaf Spot | Septoria Leaf Blotch |
|---|---|---|---|---|---|
| 24 | 11.0 | 4(2S) | 3(2S) | 1 | 4(2S) |
| | 11.0 | 5(2G) | | | 4(2S) |
| | 3.0 | 5(2G) | | | 4(2S) |
| | 3.0 | 5(2S) | | | – |
| | 0.7 | 4 | | | 3 |
| | 0.7 | 4(2S) | | | |
| | 0.2 | 3 | | | |
| 25 | 11.0 | 1 | 1 | 1 | 1 |
| 26 | 11.0 | 3 | 1 | 1 | 4(2C) |
| | 11.0 | | | | 4(2C) |
| | 3.0 | | | | 1 |
| | 0.7 | | | | 1 |
| 28 | 11.0 | 1 | 1 | 1 | 1 |
| 29 | 11.0 | 4(2GS) | 1 | 1 | 5(3GS) |
| | 11.0 | 4(2GS) | | | 5(2GS) |
| | 3.0 | 4(2S) | | | 4(2G) |
| | 0.7 | 3 | | | 1 |

Experiment 7

Certain compounds employed in the present invention have been further evaluated in soil disease tests to demonstrate their antifungal activity. The compound was formulated by dissolving 57 mg. of compound in 1 ml. of a fifty percent (v/v) solution of acetone and ethanol. A 0.1% aqueous solution of Tween 20 was added to bring the final volume to 16 ml., which was equivalent to 40 pounds/acre.

Pathogen-infested soil was placed in 8 oz. paper cups. A depression was made in the surface of the soil and 3 g. of Celatom MP-78 granules were placed in the depression. A 4 ml. aliquot of chemical formulation was added to the granules, and the cups were then covered with lids. The containers were shaken by hand for about 10 seconds, and then placed on a roller for about 10 minutes to thoroughly incorporate the test chemical into the soil. The treated soil was transferred to a 2.5 inch round plastic pot, and seeds of the host plant were added, and covered with additional treated soil. The pathogens and their corresponding host plants employed as follows:

| | | |
|---|---|---|
| Rhizoctonia | - | Cotton |
| Pythium | - | Cotton |
| Fusarium | - | Bean |
| Verticillium | - | Cotton |

The effect of the compound was observed on the growing plants and was rated on a scale of 1 to 5 (1 is severe disease, 5 is no disease). The results of such evaluations are presented in Table 9.

Table 9

| Example No. of Compound Tested | Application Rate (lbs./acre) | Rhizoctonia Damping-Off | Pythium Damping-Off | Fusarium Root Rot | Verticillium Wilt |
|---|---|---|---|---|---|
| 3 | 40.0 | 4(3S) | 1(3S) | 1(4BS) | 1 |
| 4 | 40.0 | 4(3BS) | 1 | 1(4BS) | 1 |
| 5 | 40.0 | 1 | 1 | 1 | 1 |
| 6 | 40.0 | 5(4B) | 1 | 5(4S) | 1 |
| | 40.0 | 4(4B) | | 5(4B) | |
| | 20.0 | 5(4B) | | 5(4B) | |
| | 10.0 | 5 | | 1(3S) | |
| | 10.0 | 4 | | | |
| | 5.0 | 4 | | | |
| | 2.5 | 1 | | | |
| 11 | 40.0 | 3(3S) | 1 | 1(4BS) | 1 |
| 15 | 40.0 | 4(4S) | 1 | 1(4BS) | 1 |
| 16 | 40.0 | 1 | 1 | 1 | 1 |
| 18 | 40.0 | 1 | 1 | 5(3SF) | 1 |
| 19 | 40.0 | 1 | 1 | 3 | 1 |
| 20 | 40.0 | 1 | 1 | 3 | 1 |
| 21 | 40.0 | 1 | 1 | 3(3SF) | 1(2G) |
| 22 | 40.0 | 4(2S) | 1 | 3(3SF) | 1 |
| 26 | 40.0 | 4 | 1 | 5(4SF) | 1 |
| 27 | 40.0 | 4 | 1 | 1 | 1 |
| 28 | 40.0 | 1 | 1 | 4 | 5 |
| 29 | 40.0 | 4(4S) | 1 | 5 | 5(2S) |

Experiment 8

Certain of the compounds employed in the present invention were tested in this screen in order to evaluate the compounds' ability to control cucumber powdery mildew by fumigant action.

Test compounds were formulated by dissolving them in alcohol. A saturated cellulose disc of the formulated compound, having a test compound concentration of 50 mcg./disc, was affixed to an aluminum foil disc placed on a cucumber leaf. The leaf was inoculated with powdery mildew conidia (Erysiphe cichoracearum) and disease symptoms were recorded after 7 days. The data appearing below in Table 10 presents the percent of leaf area without powdery mildew infestation.

Table 10

Fumigant Action

| Example No. of Compound Tested | Percent of Leaf Area Without Powdery Mildew Infestation |
|---|---|
| 3 | 89 |
| 4 | 12 |
| 5 | 0 |
| 6 | 55 |
| 11 | 68 |
| 15 | 78 |
| 16 | 2 |
| 17 | 55 |
| 18 | 3 |
| 19 | 0 |
| 20 | 0 |
| 21 | 0 |
| 22 | 53 |
| 26 | 0 |
| 29 | 60 |

For use as fungicides or aquatic and terrestrial plant growth regulators the compounds employed in the present invention may be formulated with a suitable agriculturally- or aquatically-acceptable carrier depending on the compounds' anticipated use. Such compositions will contain from about 0.1 to about 95.0 percent by weight of the active ingredient, depending on the composition desired. Preferred formulations will contain from about 1 to about 50 percent active ingredient. Sprayable formulations are preferred, because of the rapidity and economy of application.

The most convenient formulations contemplated are in the form of concentrated compositions. Such formulations are diluted with water, generally at or near the site of application and are applied by spraying the resulting water dispersion or emulsion. The diluted compositions generally will contain the active compounds in the range from about 0.1 percent to about 10 percent by weight. Water-dispersible or emulsifiable compositions may be either solids, usually known as wettable powders, or liquids, usually known as emulsifiable concentrates or aqueous suspensions.

A typical wettable powder comprises an intimate mixture of a compound employed in the invention, an inert carrier, and one or more surfactants. The concentration of the active compound is usually from about 5 percent to about 90 percent by weight, ideally about 10 to about 70 percent. The inert carrier is usually chosen from among the attapulgite clays, the montmorillonite clays, the diatomaceous earths, the purified silicates, or other similar substances that are readily available. Effective surfactants, comprising from about 0.5 percent to about 10 percent by weight of the wettable powder, are chosen from among the sulfonated lignins, the condensed naphthalenesulfonates, the alkyl sulfates, and related materials.

A typical emulsifiable concentrate comprises from about 0.1 to about 6 pounds of a present active agent per gallon of liquid, dissolved in a mixture of an organic solvent and an emulsifier. The organic solvent is chosen with regard to its solvency and its

cost. Useful solvents include the aromatics, especially the xylenes and the heavy aromatic naphthas. Hydrophilic cosolvents such as cyclohexanone and the glycol ethers such as 2-methoxyethanol may be included. Other organic solvents may also be used, including the terpenic solvents and kerosene. Suitable emulsifiers for emulsifiable concentrates are chosen from the alkylbenzenesulfonates, naphthalenesulfonates, and nonionic surfactants such as alkylphenol adducts of polyoxyethylene, and are used at similar percentages as for wettable powders.

Dust compositions will contain a compound employed in the present invention generally in an amount from about 0.1 to about 10 percent by weight. Dusts are prepared by intimately mixing and finely grinding the compound with an inert solid diluent or carrier such as ground montmorillonite clay, attapulgite clay, talc, ground volcanic rock, kaolin clay, or other inert, relatively dense, inexpensive substances.

Solid, granular compositions are convenient for the application of compounds employed in this invention to the soil. Granules comprise an active agent dispersed on a granular inert carrier such as coarsely ground clay of from about 0.1 to about 3 mm. particle size. The compound is most conveniently applied to the clay by dissolving it in an inexpensive solvent such as acetone and applying the solution to the sized clay in an appropriate solids mixer. The solvent is then removed by evaporation or the like.

CLAIMS

1. A compound of the formula (I)

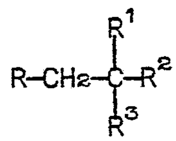 (I)

wherein:

R is 2-, 3- or 4-pyridyl, pyrazinyl, 3- or 4-pyridazinyl, or 2-, 4- or 5-pyrimidinyl;

$R^1$ is hydroxy, methoxy or ethoxy;

$R^2$ is $C_1$-$C_{10}$ alkyl, $C_3$-$C_8$ cycloalkyl, $C_1$-$C_4$ haloalkyl or

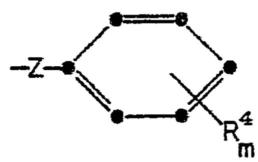 ;

$R^3$ is $C_1$-$C_{10}$ alkyl, $C_3$-$C_8$ cycloalkyl, $C_3$-$C_8$ cycloalkyl bearing one or two substituents selected from the group consisting of $C_1$-$C_4$ alkyl, halogen, $C_1$-$C_4$ haloalkyl, and $C_1$-$C_4$ haloalkoxy, or $R^3$ is

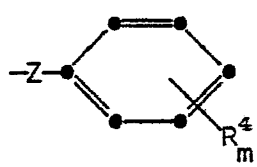 ;

$R^4$ is halogen, $C_1$-$C_4$ alkyl, $C_1$-$C_4$ alkoxy, $C_1$-$C_4$ haloalkyl or $C_1$-$C_4$ haloalkoxy;

Z is $-CH_2-(CH_2)_m-O-$ or a direct link;

each m is independently 0, 1 or 2; or

$R^2$ and $R^3$, when taken together with the carbon atom to which they are attached, form dibenzo-[A,D]cyclohepta[1,4]dienylidene, 9-fluorenylidene, 9-thioxanthenylidene, 10,11-dihydro-5H-dibenzo[A,D]-cyclohepten-5-ylidene or 1,2,3,4-tetrahydronaphthalen-1-ylidene;

or an agriculturally-acceptable salt thereof for use as an antifungal agent.

2. A compound of the formula (I)

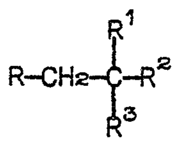

(I)

wherein:

R is 2-, 3- or 4-pyridyl, pyrazinyl, 3- or 4-pyridazinyl, or 2-, 4- or 5-pyrimidinyl;

$R^1$ is hydroxy, methoxy or ethoxy;

$R^2$ is $C_1$-$C_{10}$ alkyl, $C_3$-$C_8$ cycloalkyl, $C_1$-$C_4$ haloalkyl or

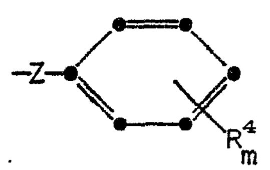

;

$R^3$ is $C_1$-$C_{10}$ alkyl, $C_3$-$C_8$ cycloalkyl, $C_3$-$C_8$ cycloalkyl bearing one or two substituents selected from the group consisting of $C_1$-$C_4$ alkyl, halogen, $C_1$-$C_4$ haloalkyl, and $C_1$-$C_4$ haloalkoxy, or $R^3$ is

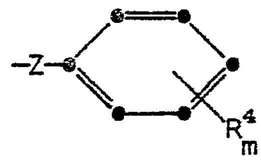 ;

$R^4$ is halogen, $C_1$-$C_4$ alkyl, $C_1$-$C_4$ alkoxy, $C_1$-$C_4$ haloalkyl or $C_1$-$C_4$ haloalkoxy;

Z is $-CH_2-(CH_2)_m-O-$ or a direct link;

each m is independently 0, 1 or 2; or

$R^2$ and $R^3$, when taken together with the carbon atom to which they are attached, form dibenzo-[A,D]cyclohexa[1,4]dienylidene, 9-fluorenylidene, 9-thioxanthenylidene, 10,11-dihydro-5H-dibenzo[A,D]-cyclohepten-5-ylidene or 1,2,3,4-tetrahydronaphthalen-1-ylidene;

or an agriculturally-acceptable salt thereof for use as an aquatic or terrestrial plant growth regulator.

3. An antifungal formulation comprising as an active ingredient from 0.1 to 95 percent by weight of a compound of formula (I):

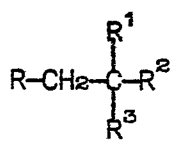 (I)

wherein:

R is 2-, 3- or 4-pyridyl, pyrazinyl, 3- or 4-pyridazinyl, or 2-, 4- or 5-pyrimidinyl;

$R^1$ is hydroxy, methoxy or ethoxy;

$R^2$ is $C_1$-$C_{10}$ alkyl, $C_3$-$C_8$ cycloalkyl, $C_1$-$C_4$ haloalkyl or

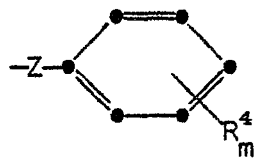 ;

$R^3$ is $C_1$-$C_{10}$ alkyl, $C_3$-$C_8$ cycloalkyl, $C_3$-$C_8$ cycloalkyl bearing one or two substituents selected from the group consisting of $C_1$-$C_4$ alkyl, halogen, $C_1$-$C_4$ haloalkyl, and $C_1$-$C_4$ haloalkoxy, or $R^3$ is

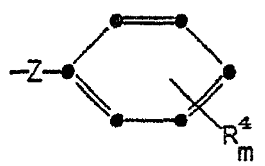 ;

$R^4$ is halogen, $C_1$-$C_4$ alkyl, $C_1$-$C_4$ alkoxy, $C_1$-$C_4$ haloalkyl or $C_1$-$C_4$ haloalkoxy;

Z is $-CH_2-(CH_2)_m-O-$ or a direct link;

each m is independently 0, 1 or 2; or

$R^2$ and $R^3$, when taken together with the carbon atom to which they are attached, form dibenzo-[A,D]cyclohepta[1,4]dienylidene, 9-fluorenylidene, 9-thioxanthenylidene, 10,11-dihydro-5H-dibenzo[A,D]-cyclohepten-5-ylidene or 1,2,3,4-tetrahydronaphthalen-1-ylidene;

or an agriculturally-acceptable salt thereof, associated with one or more agronomically acceptable carriers or diluents therefor.

4. A plant growth regulating formulation comprising as an active ingredient from 0.1 to 95 percent by weight of a compound of formula (I):

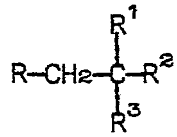 (I)

wherein:

R is 2-, 3- or 4-pyridyl, pyrazinyl, 3- or 4-pyridazinyl, or 2-, 4- or 5-pyrimidinyl;

$R^1$ is hydroxy, methoxy or ethoxy;

$R^2$ is $C_1$-$C_{10}$ alkyl, $C_3$-$C_8$ cycloalkyl, $C_1$-$C_4$ haloalkyl or

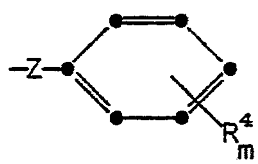 ;

$R^3$ is $C_1$-$C_{10}$ alkyl, $C_3$-$C_8$ cycloalkyl, $C_3$-$C_8$ cycloalkyl bearing one or two substituents selected from the group consisting of $C_1$-$C_4$ alkyl, halogen, $C_1$-$C_4$ haloalkyl, and $C_1$-$C_4$ haloalkoxy, or $R^3$ is

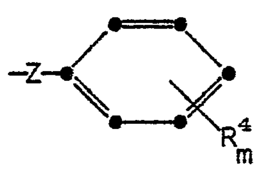 ;

$R^4$ is halogen, $C_1$-$C_4$ alkyl, $C_1$-$C_4$ alkoxy, $C_1$-$C_4$ haloalkyl or $C_1$-$C_4$ haloalkoxy;

Z is $-CH_2-(CH_2)_m-O-$ or a direct link;

each m is independently 0, 1 or 2; or

$R^2$ and $R^3$, when taken together with the carbon atom to which they are attached, form dibenzo-[A,D]cyclohepta[1,4]dienylidene, 9-fluorenylidene, 9-thioxanthenylidene, 10,11-dihydro-5H-dibenzo[A,D]-cyclohepten-5-ylidene or 1,2,3,4-tetrahydronaphthalen-1-ylidene;

or an agriculturally-acceptable salt thereof, associated with one or more agronomically acceptable carriers or diluents therefor.

5. A method of controlling fungal diseases which comprises applying to the locus where fungal disease control is desired an effective amount of a compound of formula I as defined in claim 1.

6. A method of controlling plant growth which comprises applying to a locus where control is desired a non-herbicidal, plant growth regulating amount of a compound of formula (I) as defined in claim 2.

7. A compound of the formula (II)

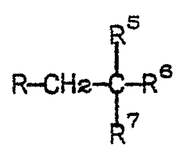

(II)

wherein:

R is 3-pyridyl, pyrazinyl, 3- or 4-pyridazinyl, or 2-, 4- or 5-pyrimidinyl;

$R^5$ is halogen, hydroxy, methoxy or ethoxy;

$R^6$ is $C_3$-$C_6$ alkyl or $C_3$-$C_6$ cycloalkyl;

$R^7$ is 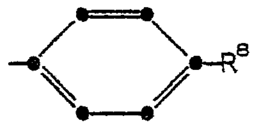 ;

$R^8$ is halogen, $C_1$-$C_4$ haloalkyl or $C_1$-$C_4$ haloalkoxy;

or an agriculturally-acceptable salt thereof.

8. A compound of Claim 7 wherein $R^1$ is hydroxy.

9. A compound selected from:

α-(2-chlorophenyl)-α-(4-chlorophenyl)-3-pyridineethanol,

α-(1-methylethyl)-α-(4-chlorophenyl)-3-pyridineethanol,

α-(1-methylethyl)-α-[4-(trifluoromethoxy)-phenyl]-3-pyridineethanol,

α-(1-methylethyl)-α-(4-chlorophenyl)pyrazine-ethanol,

α-(1-methylethyl)-α-[4-(trifluoromethyl)-phenyl]pyrazineethanol,

α-cyclohexyl-α-[4-(1,1,2,2-tetrafluoroethoxy)-phenyl]pyrazineethanol,

α-(1-methylethyl)-α-[4-(pentafluoroethoxy)-phenyl]pyrazineethanol,

α-(1-methylethyl)-α-[4-(trifluoromethoxy)-phenyl]pyrazineethanol, and

α-(1-methylethyl)-α-(4-chlorophenyl)-5-pyrimidineethanol.

10. A process for preparing a compound of the formula (II):

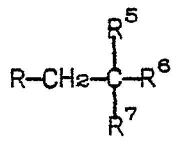 (II)

wherein:

R is 3-pyridyl, pyrazinyl, 3- or 4-pyridazinyl, or 2-, 4- or 5-pyrimidinyl;

$R^5$ is halogen, hydroxy, methoxy or ethoxy;

$R^6$ is $C_3$-$C_6$ alkyl or $C_3$-$C_6$ cycloalkyl;

$R^7$ is 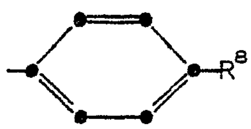 ;

$R^8$ is halogen, $C_1$-$C_4$ haloalkyl or $C_1$-$C_4$ haloalkoxy;

or an agriculturally-acceptable salt thereof;

which comprises:

(A) reacting a compound of the formula (III)

$$RCH_2M \quad (III)$$

wherein R is as defined in formula (II) and M is an alkali metal, with a compound of formula (IV)

$$R^6\overset{O}{\overset{\|}{C}}R^7 \quad (IV)$$

wherein $R^6$ and $R^7$ are as defined in formula II, in a nonaqueous organic solvent, or

(B) reacting a compound of the formula (V)

$$R-CH_2-\overset{O}{\overset{\|}{C}}-R^7 \quad (V)$$

wherein R and $R^7$ are as defined in formula (II), with a compound of the formula (VI) or (VII)

$$R^6MgX \quad (VI)$$

$$R^6M \quad (VII)$$

wherein $R^6$ is as defined in formula II, X is halogen, and M is an alkali metal, in a nonaqueous organic solvent, or

(C) reacting a compound of the formula (VIII)

$$R-CH_2-\overset{O}{\overset{\|}{C}}R^6 \quad (VIII)$$

wherein R and $R^6$ are as defined in formula (II) with a compound of the formula (IX) or (X)

$$R^7MgX \quad (IX)$$

$$R^7M \quad (X)$$

wherein $R^7$ is as defined in formula (II), X is halogen, and M is an alkali metal, in a nonaqueous organic solvent, or

(D) reacting a compound of formula (II) wherein $R^5$ is hydroxy with a halogenating reagent to provide a compound of formula (II) wherein $R^5$ is halogen, or

(E) reacting a compound of formula II wherein $R^5$ is halogen with a compound of formula (XI)

$$NaOR^{10} \quad (XI)$$

wherein $R^{10}$ is hydrogen, methyl or ethyl, to provide a compound of formula (II) wherein $R^5$ is hydroxy, methoxy, or ethoxy, or

(F) salifying a compound of formula (II).